# EUROPEAN PATENT SPECIFICATION

(11) **EP 2 623 074 B1**
(45) Date of publication and mention of the grant of the patent: **08.11.2017**
(21) Application number: 11828714.3
(22) Date of filing: 05.09.2011
(51) Int. Cl.: A61F 13/15, A61F 13/49, A61F 13/53, A61F 13/494, A61F 13/72, A61F 13/70, A61F 13/539, A61F 13/505, A61F 13/496, A61F 13/493

(54) **DISPOSABLE WEAR ARTICLE**
EINWEG-GEBRAUCHSARTIKEL
ARTICLE D'HABILLEMENT JETABLE

(30) Priority: 29.09.2010 JP 2010220023
(43) Date of publication of application: 07.08.2013
(73) Proprietor: Unicharm Corporation, Ehime 799-0111 (JP)
(72) Inventor: SASAYAMA, Kenichi, Kanonji-shi Kagawa 769-1602 (JP); ICHIKAWA, Makoto, Kanonji-shi Kagawa 769-1602 (JP); KATSURAGAWA, Kunihiko, Kanonji-shi Kagawa 769-1602 (JP)
(74) Representative: Peter, Julian
(86) International application number: PCT/JP2011/070140
(87) International publication number: WO 2012/043153

(56) References cited:
- JP-A- 2000 232 985
- JP-A- 2003 204 987
- JP-A- 2004 329 238
- JP-A- 2006 263 306
- US-A1- 2005 256 492
- US-A1- 2008 287 899

## Description

### {Technical Field}

The present invention relates to disposable wearing articles and more particularly to disposable wearing articles each provided with detachable absorbent pads, such as disposable diapers, disposable toilet-training pants, disposable incontinent pants, disposable sanitary napkins and the like.

### {Background}

Conventionally, disposable diapers provided with an absorbent pad are known. For example, JP 2006-115991 A (PTL 1) discloses a disposable wearing article provided on a skin-facing side of a diaper chassis with an absorbent pad detachably fastened thereto by fastening means.

### {Citation List}

### {Patent Literature}

{PTL 1} JP 2006-115991 A

### {Summary of Invention}

### {Technical Problem}

In the wearing article disclosed in PTL 1, an absorbent structure of the diaper is located in a front waist region and not in a rear waist region which is provided with only the absorbent pad. With such an arrangement, the crotch region should not become bulky or stiff and not create an uncomfortable feeling against the wearer, not as in the same type of the wearing article of prior art including the absorbent pad laid on the absorbent structure of the diaper.

However, if there is a size difference between the area unoccupied by the absorbent structure and the absorbent pad, i.e., if the size of the absorbent pad is smaller than that of the unoccupied area, since the absorbent pad is located in the unoccupied area, bodily fluids might not be properly absorbed in a portion which is not provided with the absorbent pad in the unoccupied area, and eventually might cling to the wearer's body. Contrariwise, if the size of the absorbent pad is larger than that of the unoccupied area, the absorbent pad might partially overlap leg-openings' peripheral gathers of the diaper chassis and cause bodily fluids to leak out sideways from the diaper chassis.
Further prior art is disclosed in US patent application US 2008/0287899 A1.

An object of the present invention is to provide a wearing article including a relatively thin bodily fluid absorbent structure extending from a crotch region into front and rear waist regions and thereby improved so that, even when a detachable absorbent pad is attached to the article, the wearing article should not create an uncomfortable feeling of stiffness against the wearer and, besides, has a preferable appearance.

### {Solution to Problem}

According to this invention, there is provided a disposable wearing article according to claim 1

According to another embodiment of this invention, an absorbent pad is detachably affixed to the skin-facing side of the bodily fluid absorbent structure.

Still another embodiment of this invention is defined in claim 3

According to yet another embodiment of this invention, a thickness dimension of the intermediate panel in its middle zone is 5.0mm or less.

According to further another embodiment of this invention, a cantilever bending resistance of the intermediate panel including the bodily fluid absorbent structure measured in the middle thereof ranges from 15 to 140mm.

### {Advantageous Effects of Invention}

In the disposable wearing article according to this invention, the intermediate panel is provided with the relatively thin bodily fluid absorbent structure and therefore the article can provide a visually neat impression without making the wearer experience a feeling of stiffness even when the detachable absorbent pad is laid on the bodily fluid-absorbent structure. In addition, at least one of the front and rear ends of the bodily fluid absorbent structure lies in the middle void spaces formed in the front and rear waist regions and, therefore, bodily fluids flowing into the middle void spaces can be quickly absorbed.

### {Brief Description of Drawings}

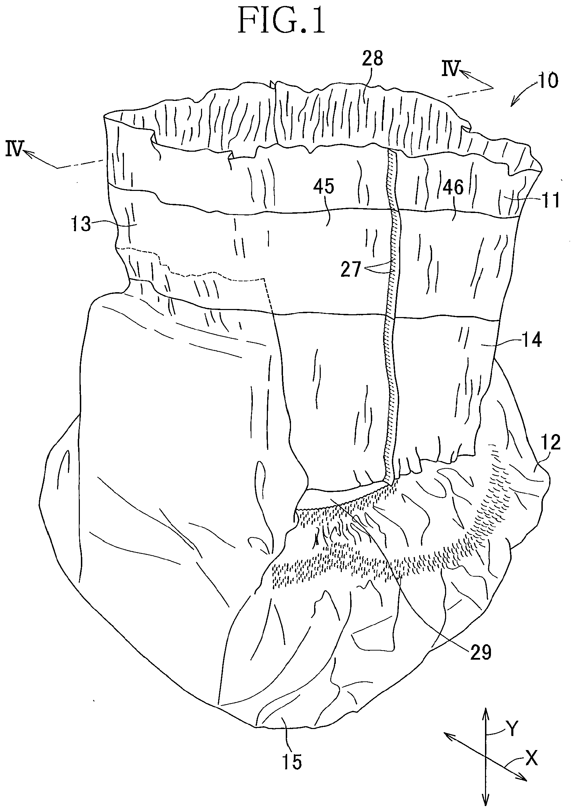

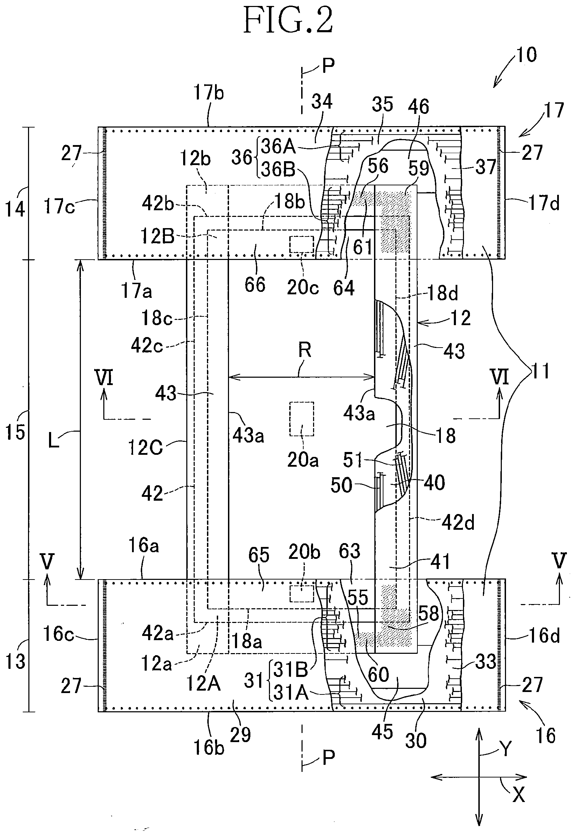

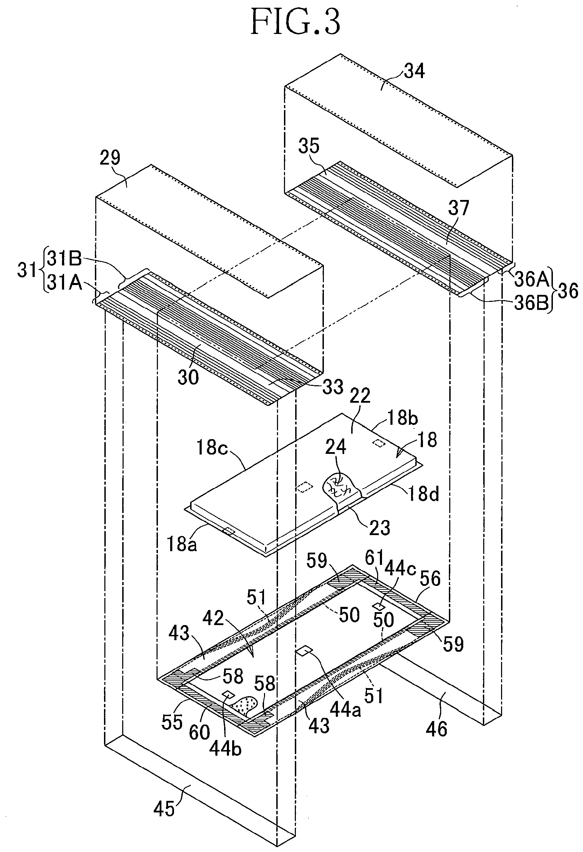

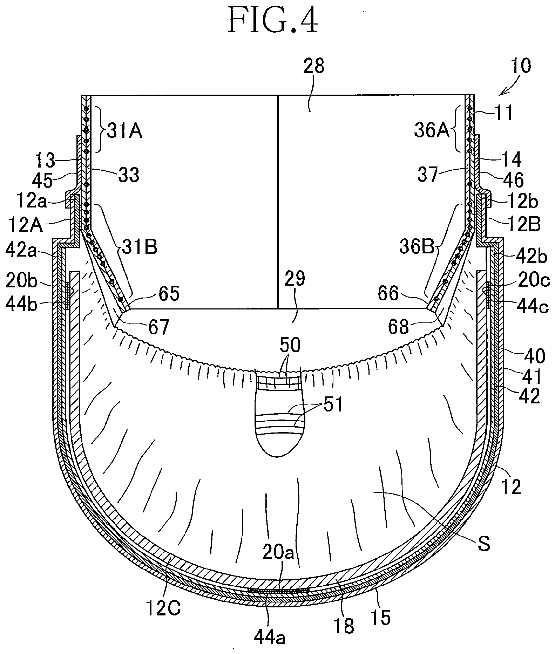

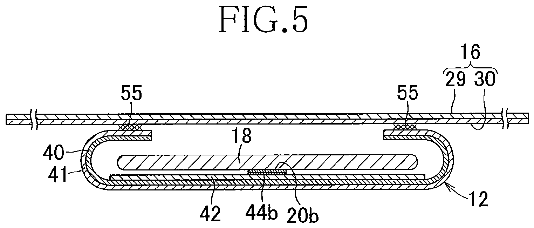

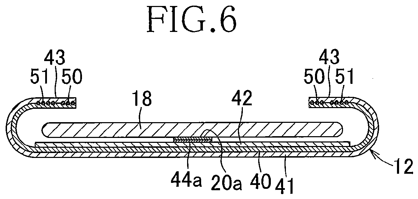

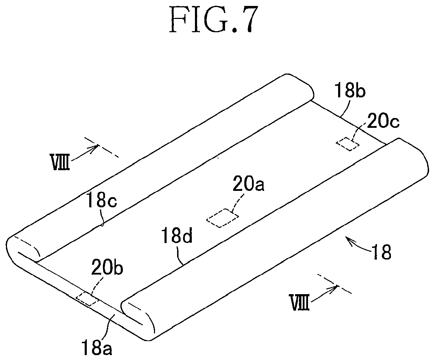

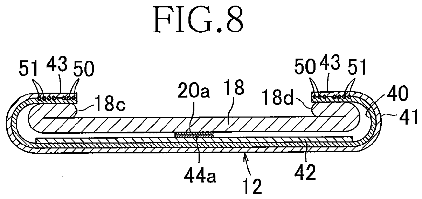

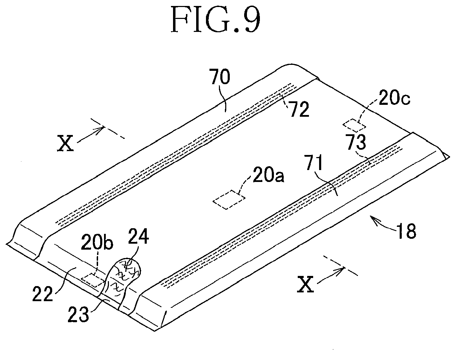

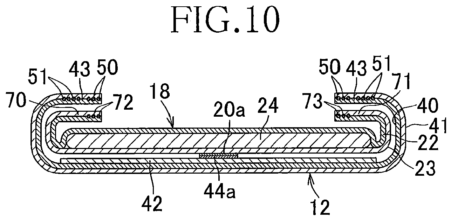

{Fig. 1} Fig. 1 is a perspective view of a disposable diaper as an example of a disposable wearing article according to a first embodiment of this invention.
{Fig. 2} Fig. 2 is a partially cutaway developed plan view showing the diaper having been developed in a longitudinal direction after side seams were released and viewed from an inner surface of the diaper.
{Fig. 3} Fig. 3 is an exploded perspective view of the diaper.
{Fig. 4} Fig. 4 is a sectional view taken along line IV-IV in Fig. 1.
{Fig. 5} Fig. 5 is a scale-enlarged sectional view taken along line V-V in Fig. 2.
{Fig. 6} Fig. 6 is a scale-enlarged sectional view taken along line VI-VI in Fig. 2.
{Fig. 7} Fig. 7 is a perspective view of an absorbent pad in the diaper according to a second embodiment of this invention.
{Fig. 8} Fig. 8 is a scale-enlarged sectional view similar to Fig. 6, illustrating the second embodiment.
{Fig. 9} Fig. 9 is a perspective view of an absorbent pad in the diaper according to a third embodiment.
{Fig. 10} Fig. 10 is a scale-enlarged sectional view similar to Fig. 6, illustrating the third embodiment.

### {Description of Embodiments}

### <First Embodiment>

Referring to Figs. 1 through 3, a disposable diaper 10 illustrated therein as an example of disposable wearing articles according to this invention has a longitudinal direction Y, a transverse direction X being orthogonal thereto and an imaginary center line P-P bisecting a width dimension in the transverse direction X, and includes a skin-facing side, a non-skin-facing side opposed to the skin-facing side, an annular elastic waist panel 11, an intermediate panel 12 attached to the skin-facing side of the elastic waist panel 11, a front waist region 13, a rear waist region 14 and a crotch region 15 extending in the longitudinal direction Y between the front and rear waist regions 13, 14. The diaper 10 is configured symmetrically about the imaginary center line P-P and the elastic waist panel 11 is composed of a front waist panel 16 defining the front waist region 13 and a rear waist panel 17 defining the rear waist region 14. The diaper 10 further includes an absorbent pad 18 extending in the longitudinal direction Y on the skin-facing side of the intermediate panel 12.

The absorbent pad 18 has a non-skin-facing side being detachably attached to the skin-facing side of the intermediate panel 12 by fastening means 20a, 20b, 20c located in the front and rearwaist regions 13, 14 and the crotch region 15, respectively. Each of the fastening means 20a, 20b, 20c may be formed of a mechanical fastener having hook elements or of a coated layer of pressure sensitive adhesive. So long as the absorbent pad 18 can be reliably stabilized so that the absorbent pad 18 should not be unintentionally released from the skin-facing side of the intermediate panel 12 and not be displaced from the predetermined position, the fastening means may be formed of at least one of the fastening means 20a, 20b, 20c, particularly the fastening means 20a located in the crotch region 15.

Referring to Fig. 3, the absorbent pad 18 is contoured by front and rear ends 18a, 18b and opposite side edges 18c, 18d so as to have a substantially rectangular shape and includes a liquid-pervious inner sheet 22, a liquid-impervious outer sheet 23 and an absorbent core 24 molded fromamixture of super-absorbent polymer particles (hereinafter referred to as SAP) and wood fluff pulp into a pad shape and interposed between the two sheets 22, 23.

The front waist panel 16 is contoured by an inner end 16a intersecting with the intermediate panel 12 and extending in the transverse direction X, an outer end 16b opposed to the inner end 16a in the longitudinal direction Y and extending in the transverse direction X and opposite side edges 16c, 16d extending in the longitudinal direction Y between the inner and outer ends 16a, 16b so as to have a transversely long rectangular shape.

The rear waist panel 17 is substantially the same as the front waist panel 16 in shape as well as in size and contoured by an inner end 17a intersecting with the intermediate panel 12 and extending in the transverse direction X, an outer end 17b opposed to the inner end 17a in the longitudinal direction Y and extending in transverse direction X and opposite side edges 17c, 17d extending in the longitudinal direction Y between the inner and outer ends 17a, 17b so as to have a transversely long rectangular shape.

The side edges 16c, 16d of the front waist panel 16 and the side edges 17c, 17d of the rear waist panel 17 are put flat and joined together by a pair of series of side seams 27 formed continually in the longitudinal direction Y and thereupon a waist-opening 28 and a pair of leg-openings 29 are defined (See Fig. 1). The side seams 27 may be formed by various joining techniques of known art such as hot embossing/debossing and ultrasonic sealing.

The front waist panel 16 includes a first inner sheet 29 lying on the skin-facing side and a first outer sheet 30 lying on the non-skin-facing side. The first inner sheet 29 and the first outer sheet 30 may be respectively formed of a liquid-impervious SMS (spun bonded/melt blown/spun bonded) fibrous nonwoven fabric or a spun bonded fibrous nonwoven fabric or a plastic sheet or a laminate sheet thereof each having a mass per unit area ranging from 10 to 30g/m². These two sheets 29, 30 are joined to each other with hot melt adhesives (not shown) applied to the inner surface of at least one of these. sheets. Two or more strand or string front waist elastics 31 extending in the transverse direction X are interposed between the first inner sheet 29 and the first outer sheet 30 and attached to these sheets with hot melt adhesives (not shown) so as to be contractible in the transverse direction X. In this way, the front waist panel 16 is elasticized at least in the transverse direction X. It is also possible to bond the first inner sheet 29 and the first outer sheet 30 to each other only with hot melt adhesives applied to the respective elastics defining the front waist elastics 31.

The front waist elastics 31 composed of upper front waist elastics 31A extending along the outer end 16b of the front waist panel 16 in the transverse direction X and lower front waist elastics 31B extending along the lower end 16b in the transverse direction X. The lower front waist elastics 31B are more densely arranged than the upper front waist elastics 31A and an inelastic region 33 having none of the elastic members is defined between a predetermined pair of the adjacent upper front waist elastics 31A.

The rear waist panel 17 has a second inner sheet 34 lying on the skin-facing side and a second outer sheet 35 lying on the non-skin facing side. The second inner sheet 34 and the second outer sheet 35maybe respectively formed of an SMS fibrous nonwoven fabric or a spun bonded fibrous nonwoven fabric or a plastic sheet or a laminate sheet thereof each having a mass per unit area ranging from 10 to 30g/m². These two sheets 34, 35 are joined to each other with hot melt adhesives (not shown) applied to the inner surface of at least one of these sheets. Two or more strand or string rear waist elastics 36 extending in the transverse direction X are interposed between the second inner sheet 34 and the second outer sheet 35 and attached to these sheets with hot melt adhesives (not shown) so as to be contractible in the transverse direction X. In this way, the rear waist panel 17 is elasticized at least in the transverse direction X. The second inner sheet 34 and the second outer sheet 35 may be joined to each other only with hot melt adhesives applied to the respective elastics defining the rear waist elastics 36.

The rear waist elastics 36 composed of upper rear waist elastics 36A extending along the outer end 17b of the rear waist panel 17 in the transverse directionX and lower rear waist elastics 36B extending along the lower end 17b in the transverse direction X. The lower rear waist elastics 36B are more densely arranged than the upper rear waist elastics 36A and includes an inelastic region 37 having none of the elastic members is defined between a predetermined pair of the adjacent upper rear waist elastics 36A.

The intermediate panel 12 is substantially rectangular and has a front end portion 12A adapted to be attached to the non-skin-facing side of the front waist panel 16, a rear end portion 12B adapted to be attached to the non-skin-facing side of the rear waist panel 17, a middle portion 12C extending in the longitudinal direction Y between the front and rear end portions 12A, 12B, inner and outer crotch sheets 40, 41 at least one of which is formed of a liquid-impervious sheet, and a bodily fluid absorbent structure 42 located on the skin-facing side of the inner crotch sheet 40. The inner and outer crotch sheets 40, 41 are joined to each other with hot melt adhesives (not shown) applied to the inner surface of any one of the sheets 40, 41 wherein respective opposite lateral portions of these sheets are folded inwardly to define a pair of elastic side portions 43 to be elasticized and extending in the longitudinal direction Y on the inner surface of the bodily fluid absorbent structure 42. The bodily fluid absorbent structure 42 is provided on its skin-facing side with landing zones 44a, 44b, 44c, for example, in the form of mechanical fastener's loop elements cooperating with the fastening means 20a, 20b, 20c of the absorbent pad 18. Instead of providing the bodily fluid absorbent structure 42 with such landing zones 44a, 44b, 44c, it is also possible to affix the absorbent pad 18 directly to the surface sheet of the bodily fluid absorbent structure 42.

Cover sheets 45, 46 are attached to the front and rear end portions 12A, 12B of the intermediate panel 12 to cover them. Specifically, the respective cover sheets 45, 46 are formed of a fibrous nonwoven fabric or the like having a mass ranging from about 10 to about 30g/m² and attached to the respective non-skin-facing sides of the front and rear waist panels 16, 17 so as to overlap with the front and rear end portions 12A, 12B of the intermediate panel 12 with hot melt adhesives (not shown).

The cover sheets 45, 46 are attached to the front and rear waist panels 16, 17, respectively, so as to face the upper front and rear waist elastics 31A, 36A, more specifically, so as to face at least the inelastic regions 33, 37 of the upper front and rear waist elastics 31A, 36A, respectively. The cover sheets 45, 46 provided to cover the front and rear end portions 12A, 12B of the intermediate panel 12 in this manner advantageously serve to improve a retention strength for the intermediate panel 12 slung from the elastic waist panel 11 and, in addition, to prevent a possibility that the front and rear end portions 12A, 12B might be peeled off from the elastic waist panel 11 even if the fingers of the wearer or of an assistant personnel are unintentionally caught by the front and/or rear end portions 12A, 12B of the intermediate panel 12. When the elastic region and/or regions defined by the upper front and/or rear waist elastics 31A, 36A is or are grasped in one hand or both hands to pull the diaper 10 up to a desired level of the wearer's body, the fingers gripping the elastic region and/or regions might be caught by the inelastic region 33 and/or inelastic region 37 and the region 33 and/or region 37 might be partially broken. However, the cover sheets 45, 46 covering these inelastic regions 33, 37 sufficiently improve the sheet strength in these regions to avoid such undesirable situation.

The bodily fluid absorbent structure 42 is contoured by front and rear ends 42a, 42b and opposite side edges 42c, 42d to have a substantially rectangular shape and formed of the absorbent core made of super-absorbent polymer particles (SAP) only and wrapped with one or two liquid-pervious sheet (s) (core-wrapping sheet) made of a hydrophilic SMS nonwoven fabric or the like. Specifically, SAP are kept in evenly distributed condition by means of adhesives applied to the entire inner surface(s) of the liquid-pervious sheet(s) so as not to be displaced. Since the absorbent core of the bodily fluid absorbent structure 42 is formed of SAP alone wrapped by the liquid-pervious sheet(s), the absorbent core is correspondingly thinner than an absorbent core formed of a mixture of SAP and fluff wood pulp and, consequentially, the absorbent core formed of SAP alone has a higher capability to follow the movement of the intermediate panel 12 than that of the absorbent core formed of the mixture of SAP and fluff wood pulp. Particularly when the bodily fluid absorbent structure 42 is used with the absorbent pad 18 having been detached from the diaper 10, the absorbent pad 18 made from SAP and fluff wood pulp mixed at a mixing ratio lower than that of SAP and molded in a desired shape may be used. In this case, it is also possible to form the bodily fluid absorbent structure 42 by stacking air laid pulp replacing fluff wood pulp on the upper surface of the lower surface of SAP.

More specifically, the bodily fluid absorbent structure 42 may be formed by interposing the absorbent core between the hydrophilic SMS nonwoven fabric lying on the upper surface of the absorbent core and having a mass of about 10g/m² and the SMS nonwoven fabric lying on the lower surface of the absorbent core and having a mass of about 11g/m² and joining them together with hot melt adhesives. Alternatively, the bodily fluid absorbent structure 42 maybe divided into a plurality of collecting sections defined by bonded lines extending in the transverse direction X and the longitudinal direction Y along which the top- and outer sheets are joined to each other so that a constant and uniform quantity of SAP is distributed in each of the collecting sections. In this case, SAP is uniformly compacted in flat configuration and therefore the fluid absorbent structure 42 should not create a discomfort feeling against the wearer due to uneven distribution of SAP even if SAP absorbs bodily fluids and is swollen in the course of or after urination. Furthermore, by evenly distributing SAP, the absorption should not be disturbed by gel blocks and thereby the absorption efficiency may be improved.

As has previously been described, the bodily fluid absorbent structure 42 has a sheet configuration made of SAP only or the mixture of SAP and a relatively small quantity of fluff wood pulp, and the thickness of the intermediate panel 12 in its middle portion is about 5mm or less. And a cantilever bending resistance thereof ranges from 15 to 140mm. Such bodily fluid absorbent structure 42 is relatively thin and highly flexible in comparison with the conventional bodily fluid absorbent member. In consequence, the absorbent structure 42 allows the intermediate panel 12 to move smoothly and the bodily fluid absorbent structure 42 should not be spaced away from the intermediate panel 12 and not be displaced even when the intermediate panel 12 sags under a weight of bodily fluids received by the intermediate panel 12.

### <Thickness Measuring Method>

Thickness of the middle portion of the intermediate panel 12 was measured by the thickness measuring device (PEACOCK of OZAKI MFG. CO. LTD.) (its probe has a diameter ranging from 10 to 20mm).

### <Cantilever Bending Resistance Measuring Method>

Test pieces (length dimension in the transverse direction was 50mm and length dimension in the longitudinal direction was 150mm.) were cut off from the middle portion of the intermediate panel 12 of the diaper 10 in accordance with cantilever method specified in JIS L 1096 and the measurement was conducted on the skin-facing side as well as on the non-skin-facing side of the respective test pieces. The number of times of the measurement (n) was 3 (three).

While the diaper 10 according to this invention uses two members, i. e. , the absorbent pad 18 and the bodily fluid absorbent structure 42 laid one on another as the bodily fluid absorbent member, the absorbent pad 18 is adapted to be of the detachably attached type so that the diaper 10 may be adapted to absorb bodily fluids by the bodily fluid absorbent structure 42 with no absorbent pad 18 laid thereon.

Each of the elastic side portions 43 to be elasticized is provided with a plurality of strand or string first leg elastics 50 and second leg elastics 51 all extending in the longitudinal direction Y so that the lateral 43 may be elasticized at least in the longitudinal direction Y. The first leg elastics 50 associated with each of the elastic side portions 43 are defined by three elastics and the second leg elastics 51 associated with each of the elastic side portions 43 are defined by four elastics concavely curving in the middle portion of the crotch region 15 and rectilinearly extending toward the front and rear waist regions 13, 14. The first and second leg elastics 50, 51 are interposed between the inner and outer crotch sheets 40, 41 and attached thereto under extension in the longitudinal direction Y with hot melt adhesives (not shown) applied to the inner surface of at least one of the inner and outer crotch sheets 40, 41.

Referring to Fig. 4, with the diaper 10 put on the wearer's body, the elastic side portions 43 are spaced apart from the bodily fluid absorbent structure 42 and put in contact with the wearer's inguinal regions and the intermediate panel 12 has the form of a bag slung from the elastic waist panel 11 under the effect of the first and second leg elastics 50, 51 attached to the respective elastic side portions 43. When body waste is discharged onto the absorbent pad 18 of the diaper 10 put on the wearer's body in this manner, the absorbent pad 18 is spaced away from the wearer's buttocks under its own weight and a large body waste collecting space S, compared to conventional disposable diapers, being adapted to collect and to contain body waste is formed between the wearer's skin and the absorbent pad 18. Since this body waste collecting space S has a larger capacity than that in the conventional disposable diapers, the body waste collecting space S is capable of collecting and containing a larger quantity of body waste. In addition, the intermediate panel 12 is slung off together with the absorbent pad 18 from the wearer's buttocks and thereby to protect the wearer' s buttocks from being soiled with body waste.

Referring to Figs. 3 through 5, the intermediate panel 12 is attached to the respective outer surfaces of the front and rear waist panels 16, 17 at a front attachment zone 55 and a rear attachment zone 56 defined by hot melt adhesives applied to the respective skin-facing sides of the front end portion 12A and the rear end portion 12B. By attaching the front and rear end portions 12A, 12B of the intermediate panel 12 to the respective non-skin-facing sides of the front and rear waist panels 16, 17, it is possible to define a further enlarged body waste collecting space S. It should be appreciated that at least one of the front and rear end portions 12A, 12B may be attached to the non-skin-facing side of the front waist panel 16 or the rear waist panel 17 so long as the body waste collecting space S can ensure a desired collecting capacity.

The front and rear attachment zones 55, 56 respectively have a concave shape opening toward the crotch region 12 and respective lateral zones 58, 59 defined by the opposite elastic side portions 43 coated with hot melt adhesives and respective middle zones 60, 61 extending in the transverse direction X between the respective lateral zones 58, 59. The middle zones 60, 61 lie outboard of the area occupied by the bodily fluid absorbent structure 42 as viewed in the longitudinal direction Y and non-attachment zones 63, 64 not coated with hot melt adhesives are defined between the respective lateral zones 58, 59 and the respective middle zones 60, 61. In the respective non-attachment zones 63, 64 defined in this manner, middle void spaces 67, 68 are defined between front and rear flaps 65, 66 so as to face the non-attachment zones 63, 64 of the intermediate panel 12 and the front and rear waist panels 16, 17, respectively. The front and rear attachment zones 55, 56 may have a curved shape as a whole and the lateral zones 59 may have a staircase pattern as the lateral zones 58 are so. The middle zones 60, 61 may have various shapes such as a triangular shape.

In the diaper 10 of such construction, the front and rear ends 42a, 42b of the bodily fluid absorbent structure 42 lie in the middle void spaces 67, 68 and the opposite side edges 42c, 42d of the structure 42 lie outboard of the inner side edges 43a of the respective elastic side portions 43. Therefore, bodily fluids flowing into the middle void spaces 67, 68 can be quickly absorbed by the bodily fluid absorbent structure 42. So long as the absorbent pad 18 can be placed on the skin-facing side of the intermediate panel 12, shape and size of the absorbent pad 18 may be appropriately selected and the absorbent pad 18 may be smaller or larger than the bodily fluid absorbent structure 42 without departing from the scope of the invention. However, the bodily fluid absorbent structure 42 may be generally dimensioned to be a size larger than the absorbent pad 18 as in the present embodiment to ensure that a quantity of bodily fluids flowing outside the absorbent pad 18 can be quickly absorbed.

Placement of the absorbent pad 18 on the skin-facing side of the intermediate panel 12 may be done by turning up the opposite elastic side portions 43 and/or the middle void spaces 67, 68, inserting the opposite side edges 18c, 18d and/or the front and rear ends 18a, 18b into the body waste collecting space S and partially bending the absorbent pad 18 so as to be fully set in the body waste collecting space S. Additionally, the fastening means 20a, 20b, 20c formed on the lower surface of the absorbent pad 18 may be fastened to the associated landing zones 44a, 44b, 44c formed on the bodily fluid absorbent structure 42 to stabilize the absorbent pad 18 in the body waste collecting space S and thereby to prevent the absorbent pad 18 from being displaced during use.

Even when the absorbent pad 18 has none of the fastening means 20a, 20b, 20c, the periphery of the absorbent pad 18 should not partially protrude out of the body waste collecting space S during use and/or the absorbent pad 18 itself should not be folded within the space S to the extent that the liquid-absorptive performance thereof might be unacceptably deteriorated. This is because, in the diaper 10 flatly developed with the respective elastic members not under tension but remaining contracted in the transverse direction as well as in the longitudinal direction, the opposite side edges 18c, 18d of the absorbent pad 18 lie outboard of the inner side edges 43a of the opposite elastic side portions 43 as viewed in the transverse direction X and the front and rear ends 18a, 18b of the absorbent pad 18 lie in the middle void spaces 67, 68, respectively, so that the periphery of the absorbent pad 18 is covered with an imaginary elastic belt defined by the opposite elastic side portions 43 and the front and rear flaps 65, 66. So long as such effect can be ensured, it will be allowed to cover only one of the opposite side edges 18c, 18d and only one of the front and rear ends 18a, 18b of the absorbent pad 18 with these elastic side portions 43 and the front and rear flaps 65, 66.

By additionally attaching the absorbent pad 18 to the diaper 10, the liquid-absorptive performance of the diaper 10 can be improved and a larger quantity of body waste can be absorbed and contained in comparison with the case in which the absorbent pad 18 is not attached to the diaper 10. The bodily fluid absorbent structure 42 normally attached to the intermediate panel 12 is relatively thin and relatively flexible and, therefore, even after the absorbent pad 18 has been laid on the bodily fluid absorbent structure 42, the diaper 10 should not create an uncomfortable feeling of stiffness compared to when a pair of absorbent pad 18 each having a desired thickness is layered one on another, and can provide a visually neat impression.

The first and second leg elastics 50, 51 adapted to elasticize the respective elastic side portions 43 and the lower front and rear waist elastics 31B, 36B intersecting with the front and rear end portions 12A, 12B of the intermediate panel 12 may be colored in an externally recognizable manner so that an opening of the body waste collecting space S may be surrounded by such colored elastic members. In this case, these colored elastic members serve as a marker visually indicating a size and a shape of the opening of the body waste collecting space S to facilitate the diaper handling personnel to turn up the elastic side portions 43 and/or the non-attachment zones 63, 64 of the intermediate panel 12, thereby inserting the absorbent pad 18 into the body waste-collecting space S so that the opposite side edges 18c, 18d and/or the front and rear ends 18a, 18b thereof may be covered with the elastic side portions 43 and/or the non-attachment zones 63, 64 of the intermediate panel 12.

While the size and the shape of the absorbent pad 18 may be appropriately selected so long as the absorbent pad 18 can be located within the body waste collecting space S, if the absorbent pad 18 can be stably affixed to the intermediate panel 12 not by the intermediary of the fastening means 20a, 20b, 20c, the absorbent pad 18 may have its length dimension in the longitudinal direction Y within the length dimension of the opening of the bodily fluid-collecting space S in the longitudinal direction Y as the lower limit to the length dimension of the body waste collecting space S, i. e. , the spacing dimension between the middle zone 60 of the front waist panel 16 and the middle zone 61 of the rear waist panel 17 in the longitudinal direction Y. The absorbent pad 18 may have its length dimension in the transverse direction X within the length dimension of the opening of the body waste collecting space S in the transverse direction X as the lower limit to the length dimension of the intermediate panel 12 as a whole inclusive of the opposite elastic side portions 43 in the transverse direction X. If the absorbent pad 18 has a length dimension in the transverse direction and/or in the longitudinal direction Y exceeding the above-mentioned range, it is possible to set the absorbent pad 18 in the body waste collecting space S with the opposite side edges 18c, 18d and/or the front and rear ends 18a, 18b folded inwardly as a second embodiment described later.

The respective dimensions specified by this invention will be described hereunder. Referring to Fig. 2 showing the diaper 10 in its developed state, a spacing dimension R between the inner side edges 43a of the respective elastic side portions 43 in the transverse direction X ranges from 40 to 300mm and a spacing dimension L between the front waist panel 16 and the rear waist panel 17 in the longitudinal direction Y ranges from 150 to 500mm. A length dimension of the intermediate panel 12 inclusive of the opposite elastic side portions 43 in the transverse direction X in its developed state preferably ranges from 150 to 600mm in order to form the body waste collecting space S assuring a sufficient collecting capacity.

### <Second Embodiment>

Fig. 7 is a perspective view of the absorbent pad 18 in a second embodiment of the diaper 10 according to this invention and Fig. 8 is a view similar to Fig. 6, illustrating the second embodiment. The basic construction of the diaper 10 according to this embodiment except the absorbent pad 18 is substantially the same as that of the first embodiment and will not be described hereunder.

According to this embodiment, the absorbent pad 18 is set in the body waste collecting space S with the opposite side edges 18c, 18d thereof. More specifically, the absorbent pad 18 is set in the space S with the opposite side edges 18c, 18d thereof folded inwardly on the skin-facing side so as to be kept in contact with the inner surfaces of the respective elastic side portions 43. According to such arrangement, the opposite side edges 18c, 18d of the absorbent pad 18 do not extend inwardly beyond the inner side edges 43a of the opposite elastic side portions 43 and are not exposed externally as viewed in the flatly developed diaper 10. In addition, an absorptive area of the absorbent pad 18 is larger than that in the first embodiment and a correspondingly larger quantity of bodily fluids can be absorbed.

### <Third Embodiment>

Fig. 9 is a perspective view of the absorbent pad 18 in a third embodiment of the diaper 10 according to this invention and Fig. 10 is a view similar to Fig. 6, illustrating the third embodiment. The basic construction of the diaper 10 according to this embodiment except the absorbent pad 18 is substantially the same as that of the first embodiment and will not be described hereunder.

According to this embodiment, the absorbent pad 18 is provided along opposite laterals thereof with a pair of barrier cuffs 70, 71 extending in the longitudinal direction Y. The barrier cuffs 70, 71 respectively composed of the respective opposite side edges of the top- and outer sheets 22, 23 forming the absorbent pad 18 and a plurality of elastics 72, 73 contractibly attached under tension in the longitudinal direction Y to these opposite side edges. Though not shown, front and rear ends or the opposite side edges of the respective barrier cuffs formed on the absorbent pad 18 are partially attached to the inner surface of the opposite elastic side portions 43 of the intermediate panel 12. With the diaper 10 of such arrangement put on the wearer' s body, the barrier cuffs 70, 71 are contracted together with the opposite elastic side portions 43 to which the barrier cuffs are attached and rise up together with the opposite elastic side portions 43 thereby to form a pair of double walls adapted to prevent body waste from leaking sideways.

The component members of the diaper 10 are not limited to those described in this specification but the other various types of material widely used in the relevant technical field may be used without limitation. The terms "first" and "second" used in the specification and claims of the present invention are used merely to distinguish the similar elements, similar positions or the other similar means.

### {Reference Signs List}

- 10: disposable diaper (disposable wearing article)
- 11: elastic waist panel
- 12: intermediate panel
- 12A: front end portion of intermediate panel
- 12B: rear end portion of intermediate panel
- 13: front waist region
- 14: rear waist region
- 15: crotch region
- 18: absorbent pad
- 18a: front end of absorbent pad
- 18b: rear end of absorbent pad
- 18c, 18d: opposite side edges of absorbent pad
- 42: bodily fluid absorbent structure
- 43: elastic side portions
- 55: front end attachment zone
- 56: rear end attachment zone
- 63, 64: non-attachment zones
- 67, 68: middle void spaces
- X: transverse direction
- Y: longitudinal direction

## Claims

1. A disposable wearing article (10) having a longitudinal direction (Y) and a transverse direction (X) being orthogonal to the longitudinal direction, comprising:
a skin-facing side;
a non-skin-facing side;
a front waist region (13);
a rear waist region (14);
a crotch region (15) extending between the front and rear waist regions (13, 14);
an elastic waist panel (11) which includes a front waist panel (16) defining the front waist region (13) and a rear waist panel (17) defining the rear waist region (14); ; and
an intermediate panel (12) disposed between the front and rear waist panels (16, 17), wherein:
the intermediate panel (12) has a front end portion (12A) lying in the front waist region (13), a rear end portion (12B) lying in the rear waist region (14) and a bodily fluid absorbent structure (42) extending across the crotch region (15) into the front and rear waist regions (13, 14); the disposable wearing article is **characterized in that**
the front and rear end portions (12A, 12B) of the intermediate panel (12) are attached to the skin-facing side of the elastic waist panel (11) by the intermediary of front and rear attachment zones (55, 56) except middle zones of the intermediate panel (12) in the transverse direction;
in non-attachment zones (63, 64) formed in the middle zones of the intermediate panel (12), middle void spaces (67, 68) are formed between front and rear flaps (65, 66) of the front and rear waist panels (16, 17) facing the non-attachment zones (63, 64) of the intermediate panel (12) and the front and rear waist panels (16, 17);
the bodily fluid absorbent structure (42) is contoured by front and rear ends (42a,42b) and opposite side edges (42c,42d) to have a substantially rectangular shape and formed of an absorbent core of super-absorbent polymer particles only and wrapped with one or two liquid-pervious sheet(s) made of a hydrophilic SMS nonwoven fabric, wherein the super-absorbent polymer particles are kept in evenly distributed condition by means of adhesives applied to the entirely inner surface(s) of the liquid-pervious sheet(s) so as not to be displaced;
the front and rear ends (42a, 42b) of the bodily fluid absorbent structure (42) lie in the middle void spaces (67, 68); and
the article (10) further includes an absorbent pad (18) extending in the longitudinal direction (Y) on the skin-facing side of the intermediate panel (12) wherein the absorbent pad (18) is laid on the skin-facing side of the bodily fluid absorbent structure (42).

2. The wearing article defined by claim 1, wherein the absorbent pad (18) is detachably affixed to the skin-facing side of the bodily fluid absorbent structure (42).

3. The wearing article defined by claim 2, wherein front and rear ends (18a, 18b) of the (18) lie in the middle void spaces (67, 68).

4. The wearing article defined by any one of claims 1 through 3, wherein a thickness dimension of the intermediate panel (12) in its middle zone is 5.0mm or less.

5. The wearing article defined by any one of claims 1 through 4, wherein a cantilever bending resistance of the intermediate panel (12) including the bodily fluid absorbent structure (42) measured in a middle thereof ranges from 15 to 140 mm.

## Patentansprüche

1. Wegwerfbarer Trageartikel (10) mit einer Längsrichtung (Y) und einer zu der Längsrichtung orthogonalen Querrichtung (X), umfassend:
eine Haut zugewandte Seite;
eine Haut abgewandte Seite;
einen vorderen Taillenbereich (13);
einen hinteren Taillenbereich (14);
einen Schrittbereich (15), der sich zwischen den vorderen und hinteren Taillenbereichen (13, 14) erstreckt;
ein elastisches Taillenpaneel (11), das ein den vorderen Taillenbereich (13) bestimmendes vorderes Taillenpaneel (16) und ein den hinteren Taillenbereich (14) bestimmendes hinteres Taillenpaneel (17) umfasst, und
ein Zwischenpaneel (12), das zwischen dem vorderen und dem hinteren Taillenpaneel (16, 17) angeordnet ist, wobei:
das Zwischenpaneel (12) einen vorderen Endabschnitt (12A), der in dem vorderen Taillenbereich (13) liegt, einen hinteren Endabschnitt (12B), der in dem hinteren Taillenbereich (14) liegt, und eine KörperFlüssigkeiten absorbierende Struktur (42) aufweist, die sich über den Schrittbereich (15) in die vorderen und hinteren Taillenbereiche (13, 14) erstreckt; wobei der wegwerfbare Trageartikel **dadurch gekennzeichnet ist, dass**
die vorderen und hinteren Endabschnitte (12A, 12B) des Zwischenpaneels (12) an der Haut zugewandten Seite des elastischen Taillenpaneels (11) durch das Dazwischenliegen von vorderen und hinteren Befestigungszonen (55, 56), mit Ausnahme von Mittelzonen in die Querrichtung des Zwischenpaneels (12), befestigt sind;
in Nicht-Befestigungszonen (63, 64), die in den Mittelzonen des Zwischenpaneels (12) gebildet sind, Mittelleerräume (67, 68) zwischen vorderen und hinteren Klappen (65, 66) des vorderen und hinteren Taillenpaneels (16, 17) gebildet sind, die den Nicht-Befestigungszonen (63, 64) des Zwischenpaneels (12) und dem vorderen und hinteren Taillenpaneel (16, 17) zugewandt sind;
die Körperflüssigkeiten absorbierende Struktur (42) von vorderen und hinteren Enden (42a, 42b) und gegenüberliegende Seitenkanten (42c, 42d) umrahmt wird, die eine im Wesentlichen rechteckige Form haben, und aus einem absorbierenden Kern nur aus superabsorbierenden Polymerpartikeln gebildet ist und mit einer oder zwei flüssigkeitsdurchlässigen Lage(n) umwickelt ist, die aus einem hydrophilen SMS Vliesstoff hergestellt sind, wobei die superabsorbierenden Polymerpartikel mittels eines auf die gesamten inneren Fläche(n) der flüssigkeitsdurchlässigen Lage(n) verteilten Klebestoffs in einem gleichmaßig verteilten Zustand gehalten werden, sodass sie sich nicht verlagern;
die vorderen und hinteren Enden (42a, 42b) der Körperflüssigkeiten absorbierenden Struktur (42) in den Mittelleerräumen (67, 68) liegen; und
der Artikel (10) ferner ein absorbierendes Kissen (18) umfasst, das sich auf der Haut zugewandten Seite des Zwischenpaneels (12) in die Längsrichtung (Y) erstreckt, wobei das absorbierende Kissen (18) auf die Haut zugewandte Seite der Körperflüssigkeiten absorbierende Struktur (42) gelegt ist.

2. Trageartikel nach Anspruch 1, wobei das absorbierende Kissen (18) lösbar an der Haut zugewandten Seite der Körperflüssigkeiten absorbierenden Struktur (42) befestigt ist.

3. Trageartikel nach Anspruch 2, wobei vordere und hintere Enden (18a, 18b) des (18) in den Mittelleerräumen (67,68) liegen.

4. Trageartikel nach einem der Ansprüche 1 bis 3, wobei eine Dickenabmessung des Zwischenpaneels (12) in seiner Mittelzone 5,0mm oder weniger ist.

5. Trageartikel nach einem der Ansprüche 1 bis 4, wobei ein Auslegerbiegewiderstand des Zwischenpaneels (12), das die Körperflüssigkeiten absorbierende Struktur (42) umfasst, in einer Mitte davon gemessen zwischen 15 bis 140 mm ist.

## Revendications

1. Article d'habillement jetable (10) ayant une direction longitudinale (Y) et une direction transversale (X) étant orthogonale à la direction longitudinale, comprenant :
un côté tourné vers la peau ;
un côté non tourné vers la peau ;
une région de taille avant (13) ;
une région de taille arrière (14) ;
une région d'entrejambe (15) s'étendant entre les régions de taille avant et arrière (13, 14) ;
un panneau de taille élastique (11) qui comprend un panneau de taille avant (16) définissant la région de taille avant (13) et un panneau de taille arrière (17) définissant la région de taille arrière (14) ; et
un panneau intermédiaire (12) disposé entre les panneaux de taille avant et arrière (16, 17), dans lequel :
le panneau intermédiaire (12) comporte une partie d'extrémité avant (12A) située dans la région de taille avant (13), une partie d'extrémité arrière (12B) située dans la région de taille arrière (14) et une structure d'absorption de fluide corporel (42) s'étendant à travers la région d'entrejambe (15) dans les régions de taille avant et arrière (13, 14) ; l'article d'habillement jetable est **caractérisée en ce que**
les parties d'extrémité avant et arrière (12A, 12B) du panneau intermédiaire (12) sont fixées sur le côté tourné vers la peau du panneau de taille élastique (11) par l'intermédiaire de zones de fixation avant et arrière (55, 56) à l'exception de zones intermédiaires du panneau intermédiaire (12) dans la direction transversale ;
dans des zones de non-fixation (63, 64) formées dans les zones intermédiaires du panneau intermédiaire (12), des espaces annulaires intermédiaires (67, 68) sont formés entre des rabats avant et arrière (65, 66) des panneaux de taille avant et arrière (16, 17) faisant face aux zones de non-fixation (63, 64) du panneau intermédiaire (12) et des panneaux de taille avant et arrière (16, 17) ;
la structure d'absorption de fluide corporel (42) est contournée par des extrémités avant et arrière (42a, 42b) et des bords latéraux opposés (42c, 42d) pour avoir une forme sensiblement rectangulaire et formée d'un noyau absorbant de particules de polymère super-absorbantes seulement et enveloppées avec une ou deux feuille(s) perméable(s) aux liquides constituée(s) d'un tissu non tissé SMS hydrophile, dans lequel les particules de polymère super-absorbantes sont maintenues dans un état uniformément réparti au moyen d'adhésifs appliqués à la/aux surface(s) entièrement interne(s) de la/des feuille(s) perméable(s) aux liquides afin de ne pas être déplacées ;
les extrémités avant et arrière (42a, 42b) de la structure d'absorption de fluide corporel (42) se situent dans les espaces vides intermédiaires (67, 68) ; et
l'article (10) comprend en outre un tampon absorbant (18) s'étendant dans la direction longitudinale (Y) sur le côté tourné vers la peau du panneau intermédiaire (12) dans lequel le tampon absorbant (18) est posé sur le côté tourné vers la peau de la structure d'absorption de fluide corporel (42).

2. Article d'habillement selon la revendication 1, dans lequel le tampon absorbant (18) est fixé de manière amovible sur le côté tourné vers la peau de la structure d'absorption de fluide corporel (42).

3. Article d'habillement selon la revendication 2, dans lequel des extrémités avant et arrière (18a, 18b) du tampon absorbant (18) se situent dans les espaces vides intermédiaires (67, 68).

4. Article d'habillement selon l'une quelconque des revendications 1 à 3, dans lequel une dimension d'épaisseur du panneau intermédiaire (12) dans sa zone intermédiaire est de 5,0 mm ou moins.

5. Article d'habillement selon l'une quelconque des revendications 1 à 4, dans lequel une résistance à la flexion en porte-à-faux du panneau intermédiaire (12) comprenant la structure d'absorption de fluide corporel (42) mesurée en son milieu varie de 15 à 140 mm.
